# EUROPEAN PATENT APPLICATION

(11) **EP 2 500 001 A2**
(43) Date of publication of application: **19.09.2012**
(21) Application number: 10827940.7
(22) Date of filing: 05.11.2010
(51) Int. Cl.: A61F 5/56

(54) **SYSTEM AND METHOD FOR TREATING SLEEP APNOEA AND SNORING**

(30) Priority: 09.11.2009 ES 200902253; 04.11.2010 ES 201001459
(71) Applicant: Muñoz Saiz, Manuel, 04004 Almeria (ES)
(72) Inventor: Muñoz Saiz, Manuel, 04004 Almeria (ES)
(86) International application number: PCT/ES2010/000492
(87) International publication number: WO 2011/054985

(57) **Abstract**

System and method for the treatment of sleep apnea and snoring.that consists of a removable collar comprising a tubular sleeve inside which runs a band wiht a thickness between 2 and 4 cm. of foam rubber or similar material,, which is applied around the neck or a portion thereof, foam rubber band held and kept separate subject's jaw and collarbone / sternum or in which it rests, forcing the head to remain upright or extended and the muscle tissues of the throat to stay tight, the collar is divided into three main sections or zones, a) the front, in which the collar once adjusted, the band adopts a curved or angular form with a height or widening of between 8 and 10 cm.approximately, which is placed between the mandible and the sternal manubrium, b) the lateral sections, band portion and / or sheath that support and stabilizes the front section, and c) The rear section where the ends of the sheath join, the lateral sections of foam, or some extensions of tape joined to said lateral sections, using Velcro, tongue, or a elastic band. the front and / or sides sections, keep separate the chin and jaw of the sternal manubrium sternum on which it rests..

## Description

FIELD OF THE INVENTION. In orthopedic equipment for the treatment of respiratory illnesses.

STATE OF THE ART: The problem of snoring and sleep apnea continues to be a medical problem. It is produced by the displacement of soft tissue and muscular relaxation in the posterior area of the throat and presents itself principally when resting in a supine position or seated, lying back in armchairs. The sleep of a patient with sleep apnea is not refreshing and daytime drowsiness, headaches, tiredness and chronic fatigue appear as well as respiratory, cardiac and cerebral vascular alterations. Arterial hypertension, and arrythmia during sleep are common. It can also produce impotence and reduction of libido. As well as increasing the possibility of falling asleep at the wheel while driving or even at work. The problem increases when sleep apnea is associated with problems of regurgitation,and in that case the relevant postures that are adopted in bed are incompatible. There is no efficient pharmacological treatment,the mechanical devices and the expensive pressure generators used up to now are not very effective. Surgery is recommended when any injury exists. US Patent 4366815 uses a complex and uncomfortable ant-snoring device that is unsuitable for extended use and fails to resolve the problem. With the present system most of these current inconveniences are eliminated.

### OBJECT OF THE INVENTION:

To use a surgical collar that can be of reduced size that is comfortable and avoids irritation. To be simple, with only a few elements, economic, washable, very useful and effective, easy to put on and take off and has a high level of ventilation to reduce heat and permit free head and neck movement,opposing a exponential resistance to the flexing, restricting it and permitting easy breathing.

Peripheral recesses or vertical channels to be applied to both sides of the side section of the band of the surgical collar which allow upward circulation of the air trapped between the surgical collar and neck.

To reduce or avoid snoring, tiredness and fatigue brought on by a lack of sleep tributabletoapnea. Reducing work and traffic accidents.

To avoid death by sudden death syndrome brought on by apnea.

To take advantage of the device simultaneously as a support of the cannulas or applied oxygen nasal masks to the patients

### DESCRIPTION OF THE INVENTION:

The system and method for the treatment of sleep apnea and snoring of the invention consists of a removable collar comprising a tubular sleeve inside which runs a band with a thicknes between 2 and 4 cm. of foam rubber or similar material, which is applied around the neck or a portion thereof, foam rubber band held and kept separate subject's jaw and collarbone / sternum or in which it rests, forcing the head to remain upright or extended and the muscle tissues of the throat to stay tight, the collar is divided into three main sections or zones, a) The front, in which the collar once adjusted, the band adopts a curved or angular form with a height or widening of between 8 and 10 cm. approximately, which is placed between the mandible and the sternum, b) The lateral sections, band portion and / or sheath, of uniform or convergent cross-ssection, reducing its height and / or width from its junction with the front section, or from middle to the back or intermediate side, taking the form of separate wedges which support the lower jaws and the clavicles and they support and stabilizes the front section, the foam strip of this section may extend from the front to the rear section or area of union of of the ends of the lateral sections or it may be shorter than the sleeve and extend from the front section to the lateral intermediate collar, and c) The rear section where the ends of the sheath join, the lateral sections of foam, or some extensions of tape joined to said lateral sections, using Velcro, tongue, or a elastic band. The front and / or sides sections which are also stabilizers, keep separate the chin and jaw of the sternum on which it rests. The tubular sheath is ribbed cotton fabric or similar material with blends of other fibers of soft texture, soft, absorbent, comfortable and breathable to prevent irritation and skin allergies tributabletothe large time of use. The band of foam can be manufactured by molding, cutting, punching and the like. The cutting can be performed using a shelf-like contour of a horseshoe.

The tubular sheath can be detachable, at least one of its openings being retractile, or with a ring rubber for its retraction once it has been placed superimposed. This sheath can be elastic and superimpose as a second cover to surgical collars with a fixed sheath. The cover can be open longitudinally closing itself with a Velcro fastener.

A sealing imprint can be given to the band of foam rubber or it can be covered with a fine layer of synthetic, non-porous impermeable materials to avoid humidity and mites. Nontoxic materials are used and preferably a tan or a similar color to the skin. The upper inside zone of the front section and / or the sides may be chamfered, angled or curved to fit ergonomically into the area below the chin and / or mandible.

The band of the surgical collar can be polyurethane foam rubber or a similar flexible or elastic material, semi-rigid, or with a rigid core and a soft casing of elastic foam rubber material. The band can have the form of a semi-circular portion with its lateral lengths convergent towards the extremes. The band of foam rubber of the surgical collar once extended and can have a symetrical plan view or can have its lateral lengths forming an angle between 160° and 180° in the form of a boomerang. This angle obliges the front section to adopt an inclination in respect to the neck to proportion a separation or chamber with the same, of one or two centimeters approximately, to airate and offer comfortability, reducing the contact zone with the skin. It can have the flat top or the bottom and rounded corners. The edges of the central section of the collar may protrude outward vertically. To facilitate aeration, the collar band edges may carry crenellated or semicircular holes or recesses in the peripheral top and bottom edges or on their inner edges and / or vertical grooves, particularly in the lateral sections, which provide the chamber between the neck and a band ascending currents. The inner face of the band may be convex and can add an internal longitudinal prominence. Can also carry in the lateral sections some triangular ventilation holes, reducing the consistency, weight and skin contact. Flexible collar allows all movements except flexing, to which it opposes more resistance or limitation

The front section can have a recess to accommodate the chin or to prevent this to support in this section.

The front and / or side sections cross-section can be rectangular, trapezium or trapezoid and its walls may be flat or curved. The curved walls may be concave or convex inward. Both reduce skin contact.

The superior zone of the front length can support itself: a) In the medium zone of the jaw: b) In the anterior zone of the same: c) In the anterior zone partially surrounding the chin and in the digastric and mylohyoid muscle and d) In all the inferior zone of the jaw. In every case the inferior zone of the front section supports itself in the external manubrium.

The support of the front section can exclusively be effected in the digastric muscle and mylohyoid when it comes to flaccid faces.

In a variant the support of the jaw is effected principally or exclusively in the lateral sections. In this case the superior zone of the front section must be lower or be recessed. The front section or its internal zone can have an angle of 60° or 70° similar to the inferior zone of the jaw to facilitate eiration.

The band of foam rubber can carry an integrated hoop or in the most external zone or between two bands of foam rubber, that gives it the curved form or desired angle or avoids compression and displacement of the chin towards the sternum. Giving the surgical collar an internal perimeter of one or two centimetres superior to the neck of the patient allows looseness and eiration.

In hybrid or semi-rigid surgical collars the band of foam rubber can carry in its interior a plaque or plastic core with large orifices. A plaque can also be utilized between the two adhered layers of foam rubber. Polyurethane foam rubber of low, medium or high density or similar material is used, depending on the system used.

The band can carry some inner vertical channels of airation. The channels can be partial and of semi-conical form, extending themselves from the superior edges and inferior interiors to the middle zone or its proximity.

In all cases the lateral lengths stabilize and support the front length maintaining it in its position independently of the lateral movements,of flexing and extension of the head. The Velcro fastener is adjustable in agreement with the physical or personal characteristics of each patient, in that at one extreme what is not required is cut. The adjustment or separation allows a chamber or corresponding front cavity to be created between the surgical collar and the neck. The surgical collars can be supplied with different measurements, such as the height or outline. The Velcro fastener, clasps, elastic tape or the dovetailing can also be applied at the lateral sections.

On the inside of the posterior band can carry one or more protruding elements or projections of discomfort, consisting of spheres, cylinders, half cylinders or triangular prisms, wedges or ridges semi-soft or semi-rigid material with rounded corners. It can be high density foam covered with a rubber or cloth. The collar is placed so that the inner edges of the prisms rest on the nape or back of the neck and occipital area. May also consist of a circular band wholly or partially elastic which is placed in the posterior internal zone outstanding elements or projections, consisting of spheres or triangular prisms, wedges or ridges semi-soft or semi-rigid material with rounded corners. It can be high density foam with a rubber or cloth cover. When the band is partially elastic adds a further piece of cloth in series. The collar is placed so that the inner edges of the prisms or wedges rest on the nape or back of the neck and occipital area..

It carries a semi-cylindrical or prismatic core element more width or height, and takes the form of a cross. The protruding elements can be placed in the outer zone of the elastic or fabric.

The protruding elements can join by an additional band of semi-rigid material forming a single piece.

Another variant shows the front and side section formed by a plate with recesses at their edges spaced. The plate is covered in the area of the recesses with a ring of foam or soft material covered with a cloth cover. Instead of the recesses can be placed a spacer ring between the rings main. The spaces are used for aeration. The following section is an elastic band or Velcro fabric with a union.

The measurements mentioned are approximate, and not limiting, since for some patients should be increased or reduced.

The collar can carry a smooth outer sleeve trim or embossed to simulate a neck ring or cotton scarf and you can use artificial tissues formed by microfibers.

### BRIEF DESCRIPTION OF THE DRAWINGS

Figure 1 shows a schematic, side, partial and cross-section of a device with the system of the invention.
Figures 2, 20 to 23 and 25 show schematic, side views of variant of the system device of the invention arranged on a user.
Figures 3, 4, 5 and 24 show schematic, side, partial and cross-section views of devices of the invention.
Figures 6, 7 and 8 show schematic, elevation views of flexible collars and band-shaped variants of the invention.
Figures 9 and 10 and 11 show schematic and plant views of flexible collars and band-shaped flexible extended variants of the invention.
Figures 12 through 19 show schematic cross-section views of flexible forward cross-sections of the collars, applying equally to the sides of the collar.
Figure 24 shows a plan view of an alternative collar.
Figures 26 and 27 show perspective views of sleeves with collars trims.

### MORE DETAILED DESCRIPTION OF THE INVENTION

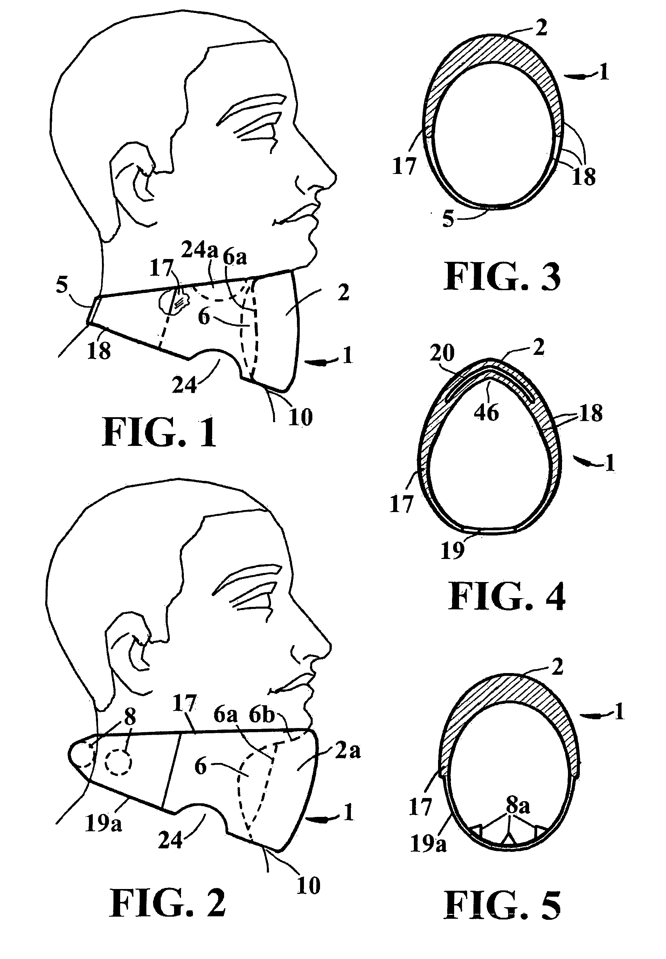

The invention, figure 1, shows the surgical elastic foam collar (1), with the convergent lateral sections (17) of short length, between the jaw and the collarbone, with its length or front zone (2) placed between the chin and the external manubrium (10) on which it rests, and as a consequence the head is erected or inclined backwards, creating the chamber or cavity (6) between the anterior face (6a) of the front section and the neck. The posterior zone is narrower and is constituted by the two ends of the material cover(18)and the Velcro union (5).Optionally the lateral sections can carry in their superior and inferior zone the recesses (24 and 24a) for aeration. The band of foam rubber can be covered with a fine isolating, anti-humidity, anti-mites layer. A detachable oversheath can be added.
Figure 2 shows the surgical collar of elastic foam rubber (1) the convergent lateral lengths (17) between the jaw and the collarbone, with their section or front zone inclined (2a) and slanted superiorly (6b) placed between the chin and the external manubrium (10) on which it supports itself, and as a consequence the head inclined backwards, creating the chamber or cavity (6) between the anterior face (6a) of the front section or zone and the neck. The back is made up of a elastic band (19a) that carries in its inner zone a few protruding elements constituted by beads (8) formed of one or more spheres with diametrical interns holes for attachment to the band by sewing. These spheres are supported in the nape or the back of the neck. Optionally the side sections can carry the recesses (24) for aeration.
Figure 3 shows the foam rubber surgical collar (1), enlarged in its central zone for major resistance or opposition to the flexing, the lateral sections (17) of short length and convergent towards its ends,vertical and/or laterally, the front section (2) and the Velcro tape (5) at the union of the ends of the cover (18). It is similar to the surgical collar of figure 1.
Figure 4 shows the foam rubber surgical collar (1), the convergent lateral lengths (17) united by means of elastic tape (19), the tubular material cover (18) the front section (2), and the internal steel strip (20) in the front and partial section of the laterals of the surgical collar giving the internal angular shape (46), this angle allows air circulation. This steel strip can also be elliptical.
Figure 5 shows the foam collar (1), with its length or front region (2), the lateral sections (17) whose ends are joined by the elastic band (19a) carrying detached or stitched to the inner area of the rear leg triangular prisms (8a), these prisms can be spaced and attached to an additional band forming a unique and rest on the neck, back of the neck or occipital area.
Figure 5 shows the foam collar (1), with its length or front region (2), the lateral sections (17) whose ends are joined by the elastic band (19a) carrying detached or stitched to the inner area of the rear leg triangular prisms (8a), these prisms can be spaced and attached to an additional band forming a unique and rest on the nape, back of the neck or occipital area.

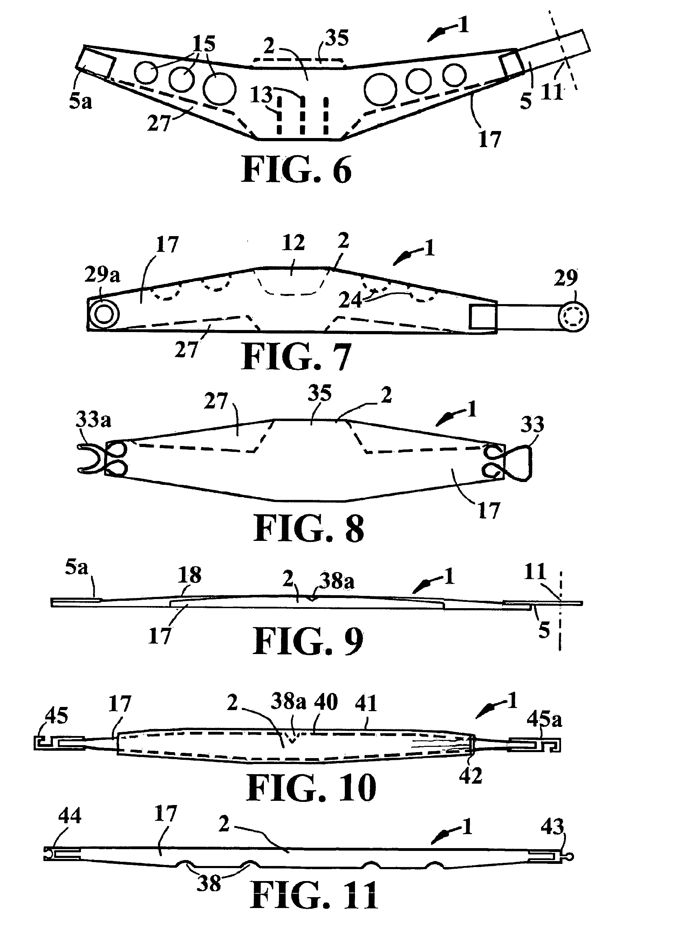

Figure 6 shows the foam rubber surgical collar (1), the convergent lateral lengths (17) with the top recess and with the optional central projection (35), when this is narrow only supports itself superiorly in the digrastic muscles and mylohyoid, he inferior edge extends downwards with the optional recesses (27) and the orifices of weight reduction and airation (15), the front zone (2) with internal reinforcing whales or bones (13) in the lower zone and the junction ends with Velcro tape (5 and 5a), and the line (11) that represents the cutting area after adjusting its length to the patient, based on their physical characteristics. It is reversible and the superior end can be interchanged with the inferior. Instead of the projection (35) may have the upper front leg recessed in order that the support is made in the lateral sections mainly or exclusively.
Figure 7 shows the foam rubber surgical collar (1) with convergent lateral sections (17), the front zone (2) with the recess (12) in its upper region, the recesses (24 and 27) in the side sectors and its ends joined by the flexible plastic clip (29 and 29a). The upper edge of the collar projecting towards said upper zone and its lower edge is flat. Is reversible, is interchangeable the top edge with the bottom one.
Figure 8 shows the foam rubber surgical collar (1) of symmetric band, the sides converging sections (17) with optional recesses (27) for aeration in the upper edges generating the projection (35) in the front region (2), and a tubular coupling (33a) with the fork (33). This collar projects from the lower edge and upper edge in its central zone. Is reversible, is interchangeable the top with the bottom.
Figure 9 shows the foam rubber surgical collar (1) with the front section (2), the converging sections formed by the cover (18) and inside the short side sections of the band (17), at the rear binding Velcro tape (5 and 5a), and the line (11) that represents the cutting area after adjusting its length to the patient based on their physical characteristics. It can have a central vertical channel external (38a). Is similar to the collar of Figure 1. The ends of the side sections can be sewing and joining with connecting elements of the back with a ribbon or strip of cloth or tarp.
Figure 10 shows the foam rubber surgical collar (1) with convergent lateral sections (17), the front (2) thickened, at the back end of union with the hooks (45 and 45a) and a removable cover or oversheath (41) with the elastic end (42). The foam coated with an insulating layer, moisture and dust mite (40) and the external central vertical channel (38a) which facilitates bending.
Figure 11 shows the foam rubber surgical collar (1) with convergent lateral sections (17) with the vertical channels of aeration (38), the front (2), and at the rear ends of the lateral sections joined by dovetailing (43 and 44). The front has the same thickness as the adjacent areas of the side sections.
   The bands of Figures 6 through 8, 10 and 11 are covered by corresponding sheaths and can have their lateral sections reduced, in that case the bonding is carried out with the ends of said sheaths.

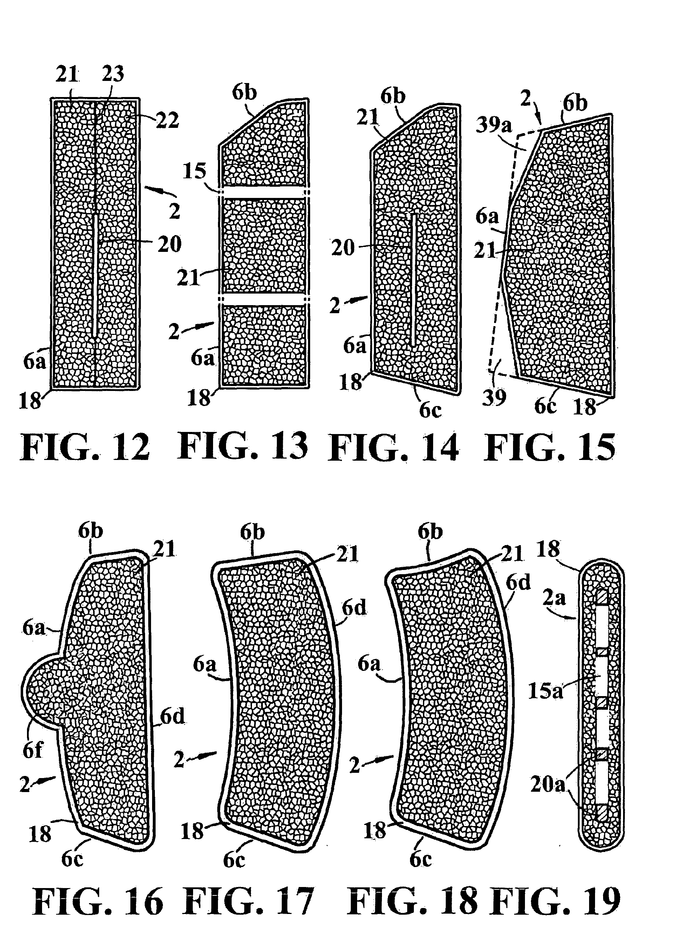

Figure 12 shows a front section (2) of rectangular section of a foam collar, two layers (21 and 22) joined by (23) with an adhesive, its front face (6a), the tubular sleeve confortable, smooth, soft and breathabl material (18) and a curve steel strip (20) reinforcing shapes, elliptical or angular to the front portion of the collar.
Figure 13 shows a front section (2) of a foam collar (21) of rectangular section with the chamfer (6b) in upper inner, its front face (6a), the aeration ducts (15), and the cover tubular (18) of soft and breathable material.
Figure 14 shows a front section (2) of a foam collar (21) of trapezoidal cross-section, with the chamfer (6b) in upper inner zone, the front face (6a), the lower face (6c), the tubular sheath soft material, soft and breathable (18) and the rod or strip (20) of reinforcement.
Figure 15 shows a front section (2) of a foam collar (21) of trapezoidal cross-section with the chamfer (6b) in upper internal zone, inclined front face (6a), the inclined bottom (6c) and the sleeve soft material, soft and breathable (18). Carries internal partial channels (39 and 39a) so semi-cónic, which extend from the upper and lower inner edges to the middle or vicinity.
Figure 16 shows a front section (2) of a foam collar (21) of plano-convex cross-section, the convex front face (6a) and the optional prominence (6f) reduce the contact with the skin and facilitates aeration, the outer face (6d) flat upper face (6b) and lower (6c) inclined and the tubular sheath of soft, breathable material (18).
Figure 17 shows a front section (2) of a foam collar (21) of curved cross-section with rounded edges, with the front face (6a) and the external face (6d), the top face (6b) and the lower (6c) inclined and the tubular sheath of soft and breathable material (18).
Figure 18 shows a front section (2) of a foam collar (21) of curved section with rounded edges, with the front face (6a) and the external face (6d), the top face (6b) inclined and curved to fit the bottom of the chin, the lower (6c) inclined and the tubular sheath of soft and breathable material(18).
Figure 19 shows a front section (2a) of a foam collar with rounded edges, with a plate or plastic core (20a), with large perforations (15a) the tubular sheath of soft and breathable material (18).

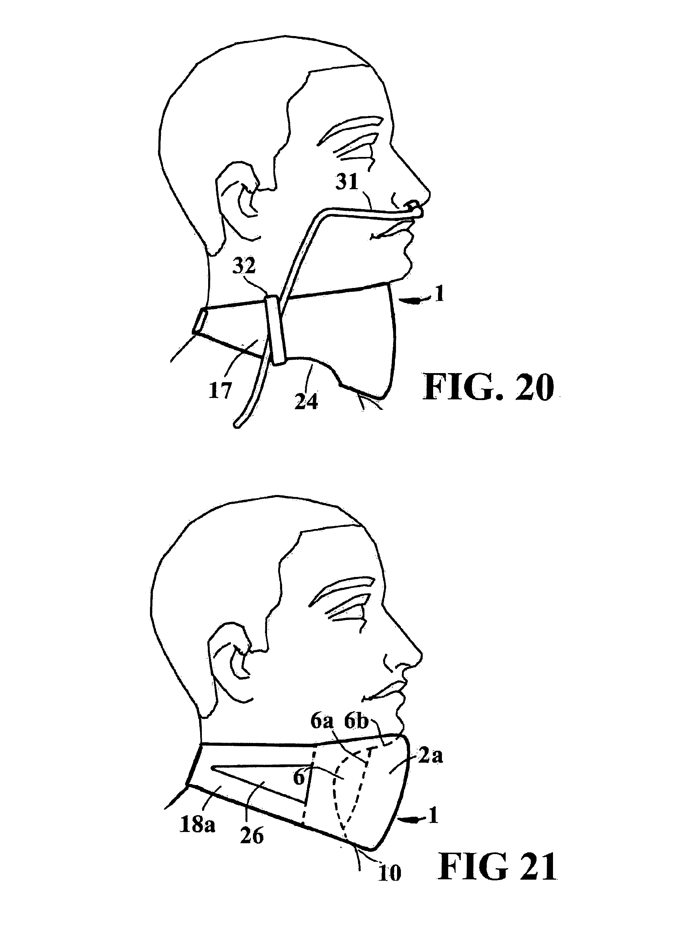

Figure 20 shows the elastic foam collar (1) with convergent lateral sections (17) between the jaw and collarbone and ventilation recess (24), having the cannula through respiration (31) secured by the clip or rubber ring (32). There may be two ducts one on each side.
Figure 21 shows the elastic foam collar (1) with its sloping front section or area (2a) and adapted superiorly (6b) to the chin and below the external manubrium (10) on which it relies, creating the chamber or cavity (6) between the anterior (6a) of the front and neck.
Part of the lateral sections and the rear are formed by the ends of the elastic circular sheath (18a) with optional aeration opening (26).

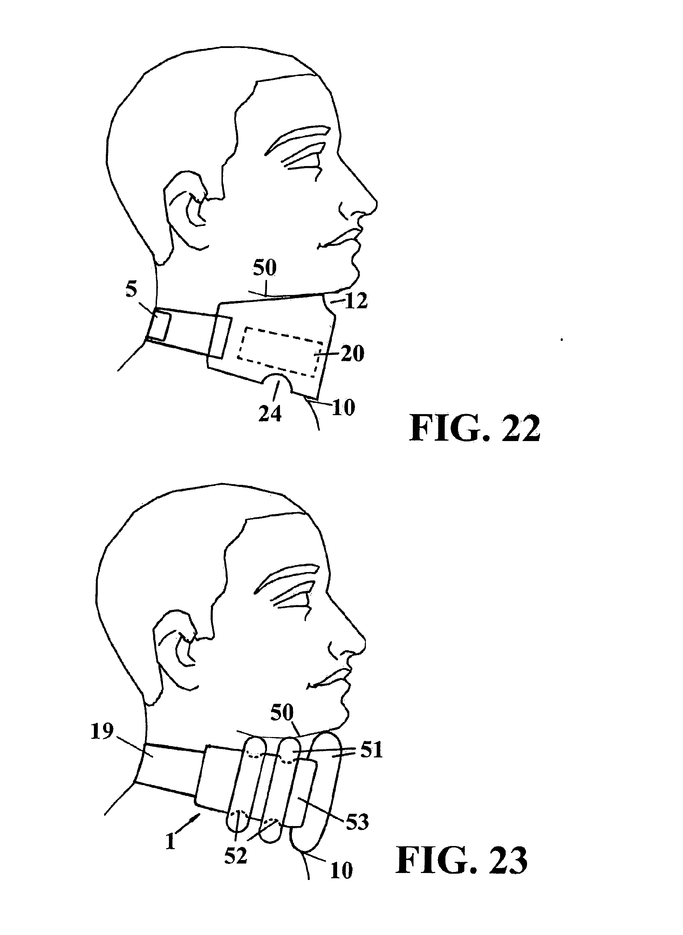

Figure 22 shows the foam collar (1) with the upper front recess (12) and the lower side (24), with binding in the back Velcro (5) and the inner reinforcing plate (20). Is placed between the jaw (50) and the external zone (10).
Figure 23 shows the collar (1) formed by the inner plate (53) covered by the foam rubber rings fabric covers (51), is tied or fastened from behind by the elastic band (19) and is supported between the jaw (53) and the external area (10).

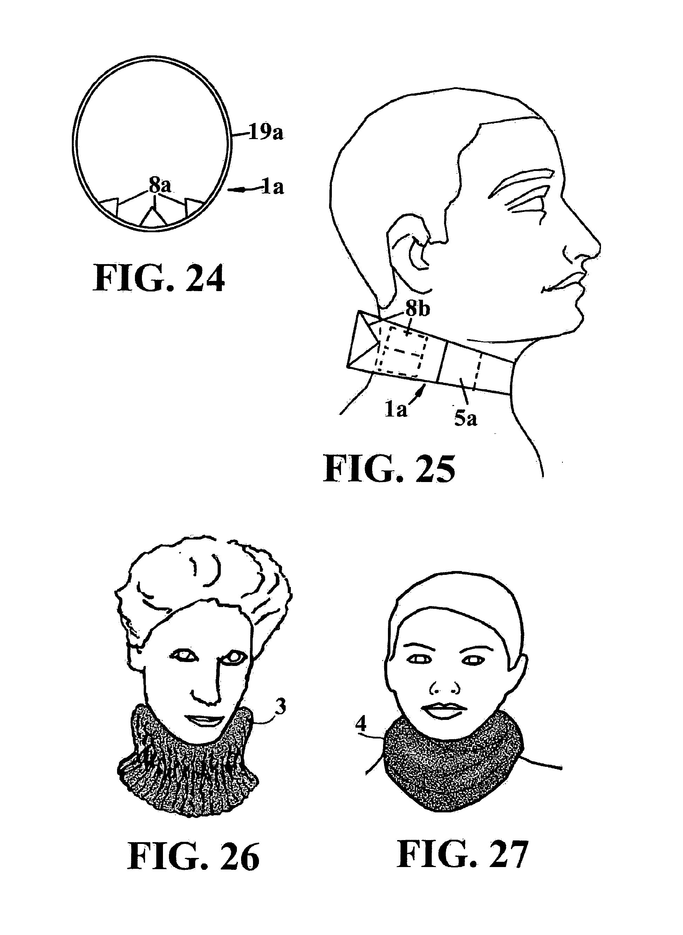

Figure 24 shows the collar (1a) formed by the elastic band (19a) with a projection or protrusions or triangular prism or wedges (8a), attached or sewn to the inner zone of the posterior segment triangular prisms (8b), such prisms can be attached to an additional band forming a single piece.
Figure 25 shows the collar (1a) formed by the band of fabric with Velcro junction (5a) the projections or triangular prism-shaped wedges (8a).
Figure 26 shows the trim cover (3) of the turtleneck collar.
Figure 27 shows the trim sheath (4) of the collar as a circular or round scarf.

## Claims

1. System and method for the treatment of sleep apnea and snoring of the type that are placed around the neck.that consists of a removable collar comprising a tubular sleeve inside which runs a band wiht a thickness between 2 and 4 cm. of foam rubber or similar material, which is applied around the neck or a portion thereof, foam rubber band held and kept separate subject's jaw and collarbone / sternum or in which it rests, forcing the head to remain upright or extended and the muscle tissues of the throat to stay tight, the collar is divided into three main sections or zones, a) he front, in which the collar once adjusted, the band adopts a curved or angular form with a height or widening of between 8 and 10 cm.approximately, which is placed between the mandible and the sternum, b) the lateral sections, band portion and / or sheath support and stabilizes the front section, the foam strip of this section may extend from the front to the rear section or area of union of of the ends of the lateral sections or it may be shorter than the sleeve and extend from the front section to the lateral intermediate collar, and c) the rear section where the ends of the sheath join, the lateral sections of foam, or some extensions of tape joined to said lateral sections, using Velcro, tongue, or a elastic band. the front and / or sides sections which are also stabilizers, keep separate the chin and jaw of the sternum on which it rests, the tubular sheath is ribbed cotton fabric or similar material with blends of other fibers of soft texture, soft, absorbent, comfortable and breathable to prevent irritation and skin allergies tributabletothe large time of use, the band of foam can be manufactured by molding, cutting, punching and the like.

2. System according to claim 1, wherein the lateral foam sections extend from the front to the intermediate side of the collar.

3. System according to claim 1, wherein the lateral foam sections extend from the front to the back or zone of union of the ends of the lateral sections.

4. System according to claim 1, wherein the upper inside zone of the front section and / or the sides may be chamfered, angled or curved to fit ergonomically into the area below the chin and / or mandible.

5. System according to claim 1, wherein the band of the surgical collar is of polyurethane foam rubber or a similar flexible or elastic material and is coated with a thin layer of non-porous synthetic and waterproof materials.

6. System according to claim 1, wherein the collar is semi-rigid with a rigid core and a soft casing of elastic foam rubber and nontoxic materials, and is used a tan or similar color to the skin

7. System according to claim 1, wherein the band of foam rubber of the surgical collar once extended has a symetrical plan view with at least one edge of the central section of the collar protruding outwards vertically.

8. System according to claim 1, wherein the band of foam rubber of the surgical collar once extended has a symetrical plan view with its lateral lengths forming an angle between 160° and 180° in the form of a boomerang, this angle obliges the front section to adopt an inclination in respect to the neck to proportion a separation or chamber with the same, of one or two centimeters approximately.

9. System according to claim 1, wherein the band of foam rubber has crenellated edges and / or semicircular holes or recesses in the peripheral top and bottom edges or on their inner edges and / or vertical grooves, particularly in the lateral sections, which provide the chamber between the neck and a band ascending currents.

10. System according to claim 1, wherein the band has the top or the bottom edge flat and/or rounded.

11. System according to claim 1, wherein the collar has an upper inner perimeter in one or two inches at the patient's neck.

12. System according to claim 1, wherein the front section of the band of foam rubber, carries inside or between two bands attached, a curved or angular plate or plastic core.

13. System according to claim 1, wherein the side sections are converged to the middle or towards the ends, reducing its height and / or thickness from its junction with the front section to an intermediate or rear zone, taking the form of wedges that support separated lower jaws of the clavicles.

14. System according to claim 1, wherein the foam rubber band is convergent from the middle of the front section to its ends, reducing its height and / or thickness adopting wedge-shaped that support separated the jaws from the clavicles.

15. System according to claim 1, wherein the front section is reinforced with a plate with large holes, grids, whales or vertical rods made from its lower edge and sewn or attached, and has a recess for accommodating the chin.

16. System according to claim 1, wherein the front section is strengthened and thickened by a triangular prism or cylindrical section of foam glued in its central inner zone which gives the collar an angular form similar to the angle of the jaw, and increases the consistency, strength or opposition to bending in that area.

17. System according to claim 1, wherein the front section is reinforced by stitching a fold in the central area of the outer band providing the mandibular angle of 60°.

18. System according to claim 1, wherein the front section is thickened in the central area.

19. System according to claim 1, wherein the cross section of the front and / or side sections is rectangular, trapezium or trapezoid, or the walls flat or curved with its corners rounded.

20. System according to claim 1 and 19, wherein the front and / or side sections add an internal longitudinal prominence.

21. System according to claim 1, wherein the upper front section rests in the middle of the jaw and the bottom of the front section rests on the sternum.

22. System according to claim 1, wherein the upper front section rests on the anterior of the jaw and lower area in the sternal manubrium.

23. System according to claim 1, wherein the upper front section rests on the anterior mandible, partly surrounding the chin, and lower area in the sternal manubrium.

24. System according to claim 1, wherein the front section is supported only superiorly in the muscles and the mylohyoid and digastric and its bottom in the manubrium.

25. System according to claim 1, wherein the upper zone of the front section rests on the entire bottom of the jaw and its bottom in the manubrium..

26. System according to claim 1, wherein the collar serves simultaneously to support the cannula that supply air or oxygen to patients through a bracket glued, stapled or sewn by its base to the cover or subject to a cord also sewn to the collar, said support consists of a flexible ring with a lateral opening for the introduction and fixation of the cannula tube.

27. System according to claim 1, wherein the collar serves simultaneously to support the cannula that supply air or oxygen to patients using a metal or plastic clip

28. System according to claim 1, wherein the support of the jaw is made primarily or exclusively in the side sections, with the upper front section lower or recessed.

29. System according to claim 1, wherein the front section of the collar has a greater thickness or central bulge in its inside.

30. System according to claim 1, wherein the front section of the collar has an external central vertical channel.

31. System according to claim 1, wherein the front section of the collar or internal middle has an angle between 60 ° and 70 ° similar to the angle formed by the lower jaw.

32. System according to claim 1, wherein the band has a steel strip integrated inside the foam band, which gives the curved shape.

33. System according to claim 1, wherein the ends of the lateral sections are sewn and attached to the attachment elements of the rear zone by a belt or band of fabric or canvas and these are joined together by buckle adjustable friction type.

34. System according to claim 1, wherein the band has a uniform section.

35. System according to claim 1, wherein the Velcro closure is adjustable according to physical characteristics or personal history of each patient, for that an end is rimmed.

36. System according to claim 1, wherein the inner zone of the elastic elements carries one or more protrusions or projections consisting of spheres, half cylinders or triangular prisms, wedges or ridges semi-soft or semi-rigid material, high density foam, with the edges rounded, the collar is positioned so that the inner edges of the prisms are supported on the nape, back of the neck and occipital region.

37. System according to claim 1, 2 and 3, wherein it is added a removable tubular sheath, which overlaps a second sheath or oversheath.

38. System according to claim 36, wherein the removable sheath carries an orifice where it enters the cannula holder.

39. System according to claim 1, wherein the collar carries a trim smooth outer sheath or embossed to simulate an annular collar or scarf.

40. System and method for treatment of sleep apnea and snoring, that consists in a detachable collar in a circular band wholly or partly elastic that is placed on the inside back or ribs protruding elements consisting of spheres or triangular prisms, wedges or ridges semi-soft or semi-rigid material with rounded edges, which are high density foam with a rubber or cloth, the collar is positioned so that the inner edges of the prisms, wedges or ridges rest on the nape or back of the neck and occipital area.

41. System and method according to claim 40, wherein the protruding elements are separated and joined by a band of semi-rigid material forming a single piece.

42. System and method according to claim 40, wherein the circular band is partially elastic in series with a complementary stretch fabric.

43. System and method according to claim 36 and 40, wherein the protruding element which is placed in the central zone is wider or higher, and takes the form of spreader.

44. System and method according to claim 36 and 40, wherein the protruding elements are placed in the outer zone of the band.

45. System and method for treating sleep apnea and snoring, that consists of a removable collar which presents the front and side section formed by a plate with recesses spaced apart at their edges, the plate is covered in the zone of the recesses with a foam rubber rings or soft material covered with a cloth cover.

46. System and method for treating sleep apnea and snoring, that consists of a removable collar which has the front and side section formed by a plate, said plate is covered in the area of the recesses with a foam rubber rings or soft material covered with a cloth cover and a spacer ring of smaller alternating between main rings, the downstream is an elastic band or fabric with a Velcro union.
